# EUROPÄISCHE PATENTSCHRIFT

(11) **EP 0 717 735 B1**
(45) Veröffentlichungstag und Bekanntmachung des Hinweises auf die Patenterteilung: **10.06.1998**
(21) Anmeldenummer: 94926244.8
(22) Anmeldetag: 07.09.1994
(51) Int. Cl.: C07D 251/34, C09K 19/34, C08G 73/06

(54) **TRIS-(CYANATO)-S-TRIAZINE, DAMIT ERHÄLTICHE ANISOTROPE DUROPLASTISCHE NETZWERKE**
TRIS-(CYANATO)-S-TRIAZINES AND ANISOTROPIC DUROPLASTIC NETWORKS OBTAINED WITH THEM
TRIS-(CYANATO)-S-TRIAZINES ET STRUCTURES RETICULAIRES THERMOPLASTIQUES ANISOTROPES REALISEES A L'AIDE DE CELLES-CI

(30) Priorität: 08.09.1993 LU 88404
(43) Veröffentlichungstag der Anmeldung: 26.06.1996
(73) Patentinhaber: EUROPÄISCHE WIRTSCHAFTSGEMEINSCHAFT (E.W.G.), 2920 Luxemburg (LU)
(72) Erfinder: MORMANN, Werner, D-57250 Netphen (DE); ZIMMERMANN, Jörg, D-57078 Siegen (DE)
(74) Vertreter: Hagemann, Heinrich, Dr.rer.nat., Dipl.-Chem.
(86) Internationale Anmeldenummer: EP9402980
(87) Internationale Veröffentlichungsnummer: WO9507268

(56) Entgegenhaltungen:
- EP-A- 0 409 069
- US-A- 5 136 011
- MACROMOLECULES, Bd.25, Nr.11, 1992, EASTON US Seiten 2947 - 2954 G.G. BARCLAY ET AL. 'Rigid-rod thermosets based on 1,3,5-triazine-linked aromatic ester segments' in der Anmeldung erwähnt
- LIQUID CRYSTALS, Bd.13, Nr.4, April 1993, LONDON GB Seiten 499 - 505 D. JANIETZ ET AL. 'Liquid crystalline aromatic esters containing a 1,3,5-triazine moiety'

## Beschreibung

Die Erfindung betrifft ein Verfahren zur Herstellung anisotroper duroplastischer Netzwerke durch Polycyclotrimerisation von Polycyanaten in der Schmelze, gegebenenfalls in der Gegenwart von Katalysatoren, Comonomeren und anderen üblichen Additiven, sowie die danach erhaltenen Verfahrenserzeugnisse.

Der relevante Stand der Technik ergibt sich beispielsweise aus der EP-A-0 409 069 (Hefner et al.). Darin werden mesogene bifunktionelle Cyansäureester beschrieben. Die Mehrzahl der darin beschriebenen Verfahrenserzeugnisse ist isotrop. Ein einziges Beispiel befaßt sich mit der Herstellung eines anisotropen Netzwerks (a.a.O., S. 66, Z. 41ff). Das danach eingesetzte Monomer in Form eines Dicyanats von 4,4'-Dihydroxybenzanilid schmilzt bei einer vergleichsweise hohen Temperatur von 184°C.

Der Erfindung liegt die Aufgabe zugrunde, Monomere bzw. zusätzliche Comonomere vorzuschlagen, mit denen stets ein anisotropes Netzwerk erhalten werden kann. Der Schmelzpunkt der Ausgangsmonomeren bzw. ihre Mischung mit den Comonomeren soll möglichst niedrig liegen, damit bei tieferen Temperaturen die Umsetzung zu den gewünchten Produkten möglich wird. Es soll auch der zusätzliche Einsatz nicht-mesogener monomerer Ausgangsmaterialien möglich sein.

Erfindungsgemäß wird diese Aufgabe durch Tris(cyanato)-s-triazine der nachfolgenden Formel (I) gelöst: 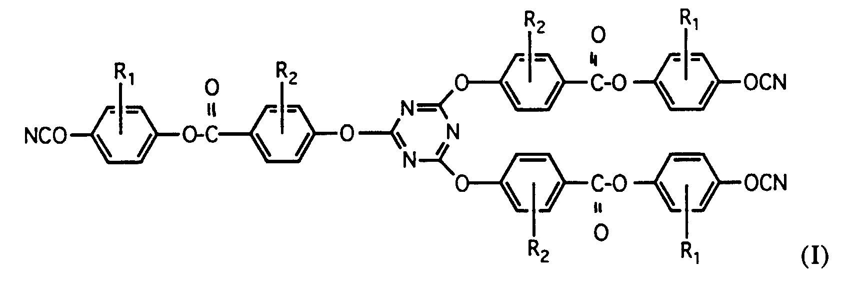 worin R₁ und R₂, jeweils unabhängig voneinander, Halogen, insbesondere Chlor, Brom und Fluor, Wasserstoff oder einen Methyl- oder Ethyl-Rest bedeuten.

Überraschend ist es für den Fachmann, daß dieses Produkt flüssigkristalline Eigenschaften aufweist, da in der Literatur ähnliche Produkte als nichtflüssigkristallin beschrieben wurden (Barday, G.G., Ober, C.K., Papathomas, K.I., Wang, D.W., *Macromolecules* **1992,**25,2974-2954).

Ferner stellt eine Lösung der obigen Aufgabe ein Verfahren der eingangs beschriebenen Art dar, das dadurch gekennzeichnet ist, daß Polycyanate in Form von flüssigkristallinen Tris(cyanato)-s-triazinen der obigen Formel (I) polycyclotrimerisiert werden.

Bei der Durchführung des erfindungsgemäßen Verfahrens erfolgt die Polycyclotrimerisation in der Schmelze. Bei den Verbindungen der obigen Formel (I) wird die Polycyclotrimerisation vorzugsweise zwischen etwa 90 bis 300°C, insbesondere zwischen etwa 120 und 220°C durchgeführt. Zweckmäßigerweise wird die Temperatur von etwa 90°C nicht unterschritten, da dann das Vorliegen kristalliner Anteile, die einer weiteren Reaktion schwer zugänglich sind, nicht ausgeschlossen werden kann. Dies würde zu einer Verschlechterung der mechanischen Eigenschaften des Verfahrensproduktes führen. Ein Überschreiten der Höchsttemperatur von etwa 300 °C führt dazu, daß der Klärpunkt der Mischung überschritten wird. Demzufolge bietet die Einhaltung des Bereiches von etwa 90 bis 300°C und insbesondere des Bereichs von etwa 120 bis 220°C Vorteile.

Das erfindungsgemäße Verfahren läßt sich ohne weiteres allein thermisch durchführen und benötigt nicht zwingend den Einsatz von Katalysatoren. Deren Einsatz beschleunigt die Polycydotrimerisation in wünschenswerter Weise und ist daher bevorzugt. Geignete Katalysatoren stellen zum Beispiel Säuren, Basen, Salze, Stickstoff- und Phosphorverbindungen dar, Lewissäuren, wie AlCl₃, BF₃, FeCl₃, TiCl₄, ZnCl₂ und SnCl_{2,} Brönstedsäuren, wie HCl und H₃PO₄, aromatische Hydroxyverbindungen, wie Phenol, p-Nitrophenol, Brenzcatechin und Dihydroxynaphthalin, Natriumhydroxid, Natriummethanola t, Natriumphenolat, Trimethylamin, Triethylamin, Tributylamin, Diazabicyclo-[2,2,2]-octan, Chinolin, Isochinolin, Tetrahydroisochinolin, Tetraethylammoniumchlorid, Pyridin-N-oxid, Tributylphosphin, Metalloctanoate, -naphthenate und -acetyl-acetonate, insbesondere in Form der entsprechenden Zink-, Zinn-, Kupfer- und Kobaltsalze. Ebenfalls als Katalysatoren sind auch Metallchelate geeignet, wie die Chelate von Übergangsmetallen und zwei- oder dreizähnigen Liganden, bevorzugt Chelate von Eisen, Kobalt, Zink, Kupfer, Mangan, Zirkon, Titan, Vanadium, Aluminium und Magnesium. Diese und andere Katalysatoren werden in den US- Patenten 3 694 410 und 4 094 852 beschrieben. Die Metallnaphthenate, -octoate und/oder - acetylacetonate werden bevorzugt, insbesondere in Form der entsprechenden Kupferverbindungen. Vorzugsweise entfallen auf 100 Gew.-Teile der reaktionsfähigen Bestandteile der monomeren Ausgangsmischung etwa 0,01 bis 3, insbesondere etwa 0,05 bis 1 Gew.-Teile Katalysator.

Zu den reaktionsfähigen Bestandteilen der Ausgangsmischung zählen neben den Tris-(cyanato)-s-triazinen der Formel (I) auch noch die nachfolgend näher erläuterten fakultativen Comonomeren. So hat es sich überraschenderweise gezeigt, daß Tris(cyanato)-s-triazine gemäß der Erfindung mit den nachfolgend näher berzeichneten nichtflüssigkristallinen Comonomeren (II) polycyclotrimerisiert werden können und dennoch ein anisotropes duroplastisches Netzwerk entsteht. Dabei kann auf die Tris-(cyanato)-s-triazine gemäß der Erfindung ein relativ hoher Anteil an nicht-flüssigkristallinem Comonomer (II) entfallen. Vorzugsweise entfallen auf 100 Gew.-Teile des Tris(cyanato)-s-triazine gemäß der Erfindung etwa 30 bis 300 Gew.-Teile, insbesondere etwa 50 bis 100 der nicht-flüssigkristallinen Comonomeren (II). Als Comonomere (II) kommen insbesondere solche der folgenden Formel (II) in Frage: 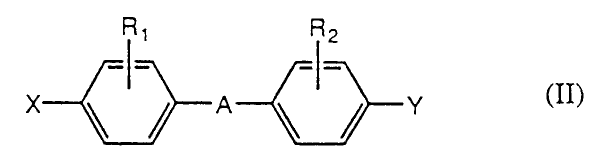 worin bedeuten:
X und Y, unabhängig voneinander, OCN oder 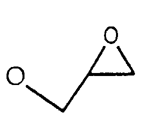
Y zusätzlich einen Alkyl-, Alkyloxy-, Alkyloxycarbonyl- oder Acyloxyrest einer Kettenlänge von 1 bis 20 Kohlenstoffatomen, gegebenenfalls. verzweigt,
A die Strukturelemente 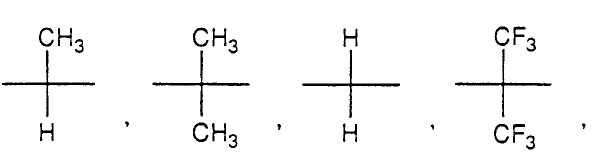 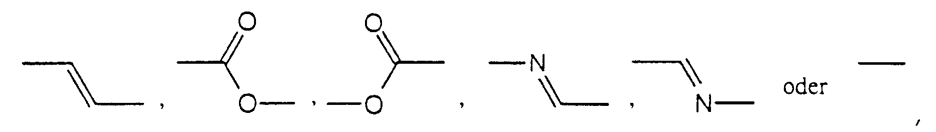 R₁ und R_{2,} unabhängig voneinander Wasserstoff, Halogen, insbesondere Chlor, Brom oder Fluor, oder einen Methyl- oder Ethylrest. Bevorzugt werden 4,4'Dicyanatophenylbenzoat, 4,4'-Dicyanatobiphenyl und 2,2-Bis(4-cyanatophenyl)propan.

Die Zumischung der nicht-flüssigkristallinen Comonomeren (II) bei der Polycyclotrimerisation bietet den Vorteil, daß anisotrope Netzwerke auch unter Verwendung beträchtlicher Mengen an billigen Monomeren, die allein nur isotrope Netzwerke ergeben, erhalten werden. Ein weiterer Vorteil der Verwendung von zusätzlichen Comonomeren (II) besteht in der Möglichkeit, die Eigenschaften des Verfahrensproduktes gezielt anzupassen, so die Vernetzungsdichte und die Erweichungstemperatur. Vorzugsweise wird zur Erzielung dieser Vorteile das Mengenverhältnis des Tris-(cyanato)-s-triazins der Formel (I) und des Comonomers (II) so gewählt wird, daß der anisotrope Charakter des angestrebten duroplastischen Verfahrenserzeugnisses gewährleistet ist.

Bei einer weiteren vorteilhaften Ausgestaltung der Erfindung werden zusätzlich andere flüssigkristalline Cyanato-Comonomere (III) mit den Tris-(cyanato)-striazinen gemäß der Erfindung allein oder zusammen mit den bereits erörterten nicht-flüssigkristallinen Comonomeren (II) umgesetzt. Als flüssigkristalline Cyanato-Comonomere (III) kommen dabei insbesondere die der nachfolgenden Formel (III) in Frage: 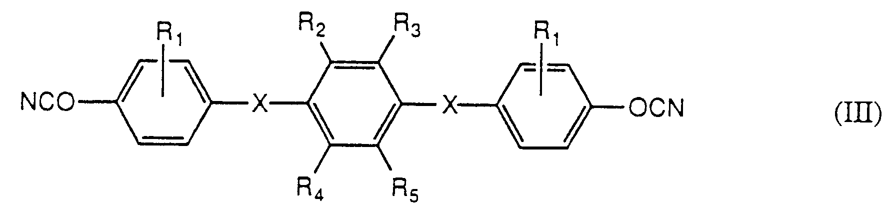 worin bedeuten:
X die Strukturelemente 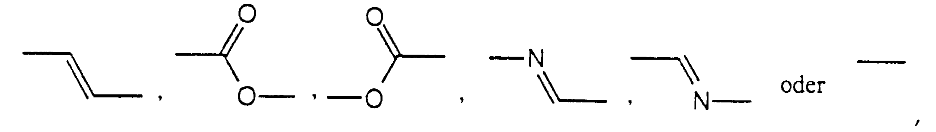
R₁ bis R_{5,} unabhängig voneinander, Wasserstoff, Halogen, insbesondere Chlor, Fluor oder Brom, einen Methyl-, Ethyl-, Propyl- oder Butylrest oder
R_{2,} R_{3,} R₄ oder R₅ einen Benzolrest,
R₂ bis R₅ Wasserstoff, wenn R₁ kein Wasserstoff ist,
R₁ Wasserstoff, wenn R₂ bis R₅ ungleich Wasserstoff sind.

Bevorzugt werden als Comonomere (III) 1,4-Bis(cyanatobenzoyloxy)benzol und 1,4-Bis(cyanatobenzoyloxy)-methylbenzol. Der erfindungsgemäße Einsatz der Verbindung der Formel (I) erniedrigt in vorteilhafter Weise den Schmelzpunkt der flüssigkristallinen Comonomere (III), die als solche nur bei Temperaturen oberhalb ihrer Schmelzpunkte, die im allgemeinen über 200 °C liegen, verarbeitet werden können.Vorzugsweise entfallen auf 100 Gew.-Teile der Tris-(cyanato)-striazine gemäß der Erfindung bis zu etwa 1000 Gew.-Teile, insbesondere etwa 100 bis 500 Gew.-Teile der Comonomere der Formel (III).

Im Rahmen der Erfindung dauert die Polycyclotrimerisation, wenn ein Katalysator eingesetzt wird, vorzugsweise etwa 0,5 bis 2 h.

Um die Eigenschaften des angestrebten duroplastischen Erzeugnisses zu verbessern, kann es in Einzelfällen vorteilhaft sein, bei erhöhter Temperatur eine Nachhärtung durchzuführen, wie es auch für isotrope Netzwerke zum Stand der Technik gehört. Beispielsweise wird 2 h bei 150 °C, 2 h bei 200 °C und weitere 2 h bei 240 °C ausgehärtet.

Um die Eigenschaften zu verbessern, kann auch während der Polycyclotrimerisation ein elektromagnetisches Feld angelegt werden (siehe z. B.in : *Finkelmann et al, Macromol. Chem. 180, 803-806 (1979):* Lc-Seitenketten enthaltende Methacrylat-Copolymere orientiert im elektrischen Feld. *Moore et al, ACS Polymeric Material Science and Engineering, 52, 84-86 (1985):* Orientierung von Seitenketten-lc-Polymeren im magnetischen Feld; *S.K. Garg, S. Kenig in: High Modulus Polymers, S. 71-103 (1988) Marcel Dekker, inc.:* Allgemeine Diskussion der Orientierung von lc-Polymeren durch Scherkräfte). Auf diese Weise werden insbesondere die thermische Ausdehnung und Zugfestigkeit in Orientierungsrichtung verbessert bzw. gesteuert.

Anhand der erfindungsgemäßen Verfahrenslehre bzw. mit den erfindungsgemäßen Tris-(cyanato)-s-triazinen läßt sich also ein vorteilhaftes anisotropes duroplastisches Netzwerk in Form eines Homo- oder Copolymerisats herstellen.

Gegenstand der Erfindung sind somit auch anisotrope duroplastische Netzwerke in Form eines Homo- oder Copolymerisats des Tris(cyanato)-s-triazins der vorstehend bezeichneten Formel (I), insbesondere in Form eines Copolymerisats der Tris(cyanate)-s-triazine der Formel (I) sowie der nicht-flüssigkristallinen Comonomeren (II) und/oder der flüssigkristallinen CyanatoComonomeren (III) gemäß den vorstehenden Definitionen. Ein vorteilhaftes Copolymerisat dieser Art zeichnet sich dadurch aus, daß darin auf 100 Gew.-Teile der Tris-(cyanato)-s-triazins der Formel (I) etwa 30 bis 300 Gew.-Teile, insbesondere etwa 50 bis 100 Gew.-Teile des Dicyanato-Comonomers (II) und/oder bis zu etwa 1000 Gew.-Teile, insbesondere etwa 100 bis 500 Gew.-Teile der flüssigkristallinen Cyanato-Comonomeren (III) entfallen.

Die Vorteile, die mit der vorliegenden Erfindung erzielbar sind, lassen sich wie folgt bezeichnen: Die Erfindung ermöglicht es, gesichert anisotrope Netzwerke herzustellen. Dabei ist sie nicht auf flüssigkristalline Ausgangsmaterialien beschränkt, sondern es können auch zusätzlich nicht-flüssigkristalline bzw. mesogene Ausgangsmaterialien eingesetzt werden. Hierdurch lassen sich die Produkteigenschaften gezielt steuern, so beispielsweise die Vernetzungsdichte mit der Folge, daß Härte, Sprödigkeit und Temperaturbeständigkeit und die Glastemperatur wünschenswert eingeregelt werden können. Damit ist auch der Vorteil verbunden, daß die angesprochenen Comonomeren mit verhältnismäßig hohem Schmelzpunkt mit den erfindungsgemäßen Tris-(cyanato)-s-triazinen aufgrund der gesenkten Schmelzpunkte der Reaktionsmischung bei wesentlich niedrigeren Temperaturen umgesetzt werden können. Darüber hinaus hat es sich gezeigt, daß die erfindungsgemäß erhaltenen anisotropen duroplastischen Netzwerke einen um den Faktor 10 gegenüber den Vergleichsprodukten des Standes der Technik verbesserten E-Modul (Maß für die Festigkeit) aufweisen.

Die erfindungsgemäßen anisotropen duroplastischen Netzwerke können nach den üblichen Verarbeitungsmethoden als Konstruktionswerkstoffe bei der Herstellung von Isolierstoffen, Laminaten, Composites, Überzügen und Beschichtungen eingesetzt werden.

Die Erfindung soll anhand nachfolgender Beispiele näher erläutert werden:

### Beispiel 1:

Herstellung der Vorstufe in Form des 1,3,5-Tris(4-hydroxyphenyloxy-4carbonylphenoxy)-2,4,6-triazins: In einen trockenen 250 ml Einhalskolben mit Schutzgasanschluß und Magnetrührstab werden 6.1 g (12.5 mmol) 1,3,5-Tris(4carboxyphenoxy)-2,4,6-triazin gegeben. Dazu gibt man 4.9 g (41.1 mmol) Thionylchlorid, eine kleine Spatelspitze 4-N,N-Dimethylaminopyridin und erhitzt die Mischung 5 Stunden auf 120 °C. Das überschüssige Thionylchlorid wird im Vakuum abgezogen. Dann gibt man 11.0 g (43.2 mmol) 1,4-Bis(trimethylsiloxy)benzol zu. Es wird eine Destillationsapparatur aufgesetzt und 12 Stunden bei 150 °C gehalten. Nach Abdestillation der berechneten Menge Chlortrimethylsilan wird das überschüssige 1,4-Bis(trimethylsiloxy)-benzol im Ölpumpenvakuum abgezogen. Das Rohprodukt wird IR-spektroskopisch identifiziert. Zur Desilylierung wird das Rohprodukt in 200 ml N,N-Dimethylformamid sowie 30 ml verdünnter Salzsäure gelöst und mehrere Stunden bei Raumtemperatur gerührt. Anschließend wird in 400 ml Wasser ausgefällt, abfiltriert, zweimal mit jeweils 100 ml Wasser gewaschen und im Vakuum bei 80 °C getrocknet. Die Identifizierung erfolgt IR-spektroskopisch.

Synthese von 1,3,5-Tris(4-cyanatophenyloxy-4-carbonylphenoxy)-2,4,6-triazin: In einem 250 ml Einhalskolben mit Magnetrührstab und Eiswasserkühlung werden 2.54 g (3.32 mmol) 1,3,5-Tris(4-hydroxyphenyloxy-4-carbonylphenoxy)-2,4,6triazin, 1.37 g (12.94 mmol) Bromcyan in 40 ml N,N-Dimethylformamid bei einer Innentemperatur von 0 - 5 °C vorgelegt. Dazu werden innerhalb 5 Minuten 1.01 g (9.96 mmol) Triethylamin zugetropft und 15 Minuten nachgerührt. Das 1,3,5Tris(4-cyanatophenyloxy-4-carbonylphenoxy)-2,4,6-triazin wird in 400 ml Eiswasser als weißer Niederschlag ausgefällt. Es wird zweimal mit jeweils 50 ml Wasser nachgewaschen, im Ölpumpenvakuum bei Raumtemperatur getrocknet und IR-spektroskopisch identifiziert. Die glasig-amorphe Verbindung erweicht bei ca. 90 °C unter Ausbildung nematischer Texturen.

### Beispiel 2:

Bildung und Charakterisierung eines anisotropen Netzwerkes aus 1,3,5-Tris(4cyanatophenyloxy-4-carbonylphenoxy)-2,4,6-triazin: Das durch Beispiel 1 gewonnene amorphe Produkt wird in einem Heiztisch von 30 °C bis 300 °C mit einer Heizrate von 20 °C aufgeheizt und in polarisiertem Licht mikroskopisch untersucht. Die bei Raumtemperatur feste Substanz wird bei 90 °C flüssig, die Schmelze zeigt die typischen Texturen einer nematisch-flüssigkristallinen Phase. Bei 200 °C nimmt die Viskosität der Mischung zu, es findet Cyclotrimerisation statt. Die Mischung wird bei ca. 250 °C fest. Die oberhalb der Erweichung erscheinenden Texturen bleiben über den ganzen Zeitraum der Härtung und auch nach Abkühlen auf Raumtemperatur erhalten. Mehrmaliges Aufheizen der Probe bis 300 °C wirkt sich nicht auf die eingefrorenen Texturen des Netzwerkes aus.

### Vergleichsbeispiel 1:

Herstellung eines Prüfstabes aus 1,3,5-Tris(4-cyanatophenyloxy-4-carbonylphenoxy)-2,4,6-triazin: Eine Probe von 1,3,5-Tris(4-cyanatophenyloxy-4carbonylphenoxy)-2,4,6-triazin wird 10 h bei 150 °C, dann 2 h bei 310 °C getempert. Der erhaltene, nicht transparente Prüfstab (23x13x2 mm) wird in Torsion untersucht (30 °C bis 400 °C, 2 °C/min, 1 Hz). Der Speichermodul G' beträgt 15,8 GPa bei 100 °C, die Glastemperatur wird bei 370 °C beobachtet.

Herstellung eines Prüfstabes aus 2,2-Bis(4-cyanatophenyl)-propan: Eine Probe von 2,2-Bis(4-cyanatophenyl)-propan wird 10 h bei 150 °C, dann 2 h bei 310 °C getempert. Der erhaltene, nicht transparente Prüfstab (23x13x2 mm) wird in Torsion untersucht (30 °C bis 400 °C, 2 °C/min, 1 Hz). Der Speichermodul G' beträgt 1.6 GPa bei 100 °C, die Glastemperatur wird bei 320 °C beobachtet.

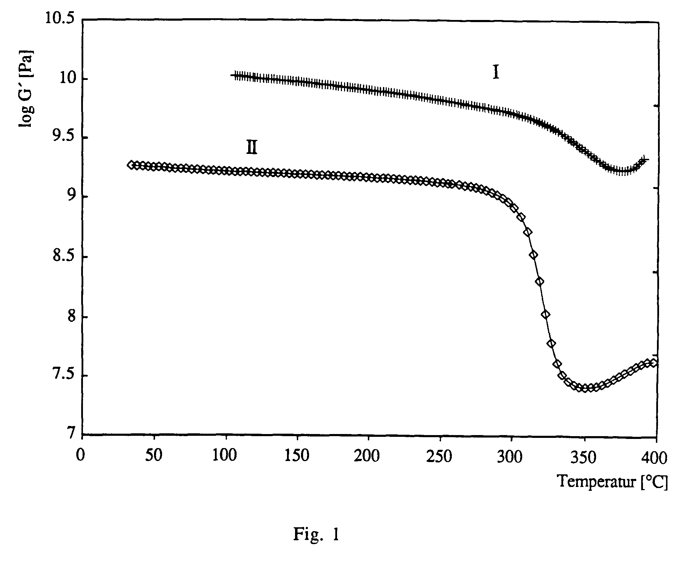

In der Figur 1 ist das Speichermodul als Funktion der Temperatur für die Prüfstäbe aus Netzwerken von 1,3,5-Tris(4-cyanatophenyloxy-4carbonylphenoxy)-2,4,6-triazin (I) und 2,2-Bis(4-cyanatophenyl)-propan (II) graphisch dargestellt (DMTA-Experiment: Heizrate 2 K/ min, Frequenz 1 Hz). Das mit dem erfindungsgemäßen Erzeugnis erhaltene Speichermodul liegt deutlich höher als das des Vergleichsproduktes.

### Beispiel 3:

Bildung und Charakterisierung eines Netzwerkes aus 1,3,5-Tris(4cyanatophenyloxy-4-carbonylphenoxy)-2,4,6-triazin mit 4,4'-Dicyanatobiphenyl: 2,0 g 1,3,5-Tris(4-cyanatophenyloxy-4-carbonylphenoxy)-2,4,6-triazin und 2,0 g 4,4'-Dicyanatobiphenyl werden homogenisiert und eine Stunde bei 180 °C sowie eine Stunde bei 250 °C gehärtet. Die nematisch-flüssigkristallinen Texturen bleiben über den gesamten Zeitraum der Härtung erhalten, auch ein Abkühlen der Probe oder ein mehrmaliges Aufheizen bis 300 °C bringt keine Änderung der anisotropen Eigenschaften.

### Beispiel 4:

Bildung und Charakterisierung eines Netzwerkes aus 1,3,5-Tris(4cyanatophenyloxy-4-carbonylphenoxy)-2,4,6-triazin mit 4,4'-Dicyanatophenylbenzoat: 1,0 g 1,3,5-Tris(4-cyanatophenyloxy-4-carbonylphenoxy)-2,4,6-triazin, 2,0 g 4,4'-Dicyanatophenylbenzoat sowie 0,15 Gew% Kupfer (II)-acetylacetonat werden homogenisiert und 15 Minuten bei 150 °C sowie eine Stunde bei 250 °C gehärtet. Die nematisch-flüssigkristallinen Texturen bleiben über den gesamten Zeitraum der Härtung erhalten. Auch ein Abkühlen der Probe oder ein mehrmaliges Aufheizen bis 300 °C bringt keine Änderung der anisotropen Eigenschaften.

### Beispiel 5:

Bildung und Charakterisierung eines Netzwerkes aus 1,3,5-Tris(4cyanatophenyloxy-4-carbonylphenoxy)-2,4,6-triazin mit 2,2-Bis(4-cyanatophenyl)propan: 2,0 g 1,3,5-Tris(4-cyanatophenyloxy-4-carbonylphenoxy)-2,4,6-triazin und 0,7 g 2,2-Bis(4-cyanatophenyl)-propan werden homogenisiert und eine Stunde bei 180 °C sowie eine Stunde bei 250 °C gehärtet. Die nematisch-flüssigkristallinen Texturen bleiben über den gesamten Zeitraum der Härtung erhalten, auch ein Abkühlen der Probe oder ein mehrmaliges Aufheizen bis 300 °C bringt keine Änderung der anisotropen Eigenschaften.

### Beispiel 6:

Bildung und Charakterisierung eines Netzwerkes aus 1,3,5-Tris(4cyanatophenyloxy-4-carbonylphenoxy)-2,4,6-triazin mit 2,2-Bis(4-epoxypropoxy)propan: 2,0 g 1,3,5-Tris(4-cyanatophenyloxy-4-carbonylphenoxy)-2,4,6-triazin, 0,4 g 2,2-Bis(4-epoxypropoxy)-propan werden homogenisiert und drei Stunden bei 130 °C, eine Stunden bei 180 °C sowie eine Stunde bei 250 °C gehärtet. Die nematisch-flüssigkristallinen Texturen bleiben über den gesamten Zeitraum der Härtung erhalten, auch ein Abkühlen der Probe oder ein mehrmaliges Aufheizen bis 300 °C bringt keine Änderung der anisotropen Eigenschaften.

## Patentansprüche

1. Tris-(cyanato)-s-triazine der Formel (I) 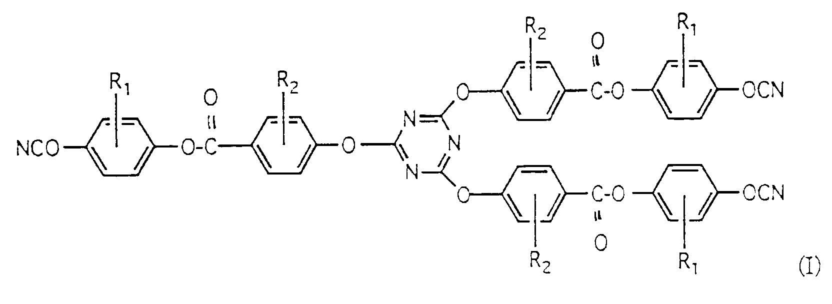 worin R₁ und R₂, jeweils unabhängig voneinander, Halogen, Wasserstoff oder einen Methyl- oder Ethyl-Rest bedeuten.

2. Verfahren zur Herstellung anisotroper duroplastischer Netzwerke durch Polycyclotrimerisation von Polycyanaten in der Schmelze, gegebenenfalls in der Gegenwart von Katalysatoren, Comonomeren und anderen üblichen Additiven, dadurch gekennzeichnet, daß Polycyanate in Form von flüssigkristallinen Tris(cyanato)-s-triazinen der Formel (I) des Anspruchs 1 polycyclotrimerisiert werden.

3. Verfahren nach Anspruch 2, dadurch gekennzeichnet, daß die Polycyclotrimerisation zwischen 90 bis 300°C durchgeführt wird.

4. Verfahren nach Anspruch 2 oder 3, dadurch gekennzeichnet, daß die Polycyclotrimerisation in Gegenwart eines Katalysators durchgeführt wird.

5. Verfahren nach Anspruch 4, dadurch gekennzeichnet, daß als Katalysator Metallnaphthenate, -octoate und/oder -acetylacetonate verwendet werden.

6. Verfahren nach einem der Ansprüche 3 bis 5, dadurch gekennzeichnet, daß auf 100 Gew.-Teile der reaktionsfähigen Bestandteile der Ausgangsmischung 0,01 bis 3 Gew.-Teile Katalysator entfallen.

7. Verfahren nach mindestens einem der Anspruch 2 bis 6, dadurch gekennzeichnet, daß Tris-(cyanato)-s-triazine der Formel (I) zusammen mit nichtflüssigkristallinen Comonomeren (II) polycyclotrimerisiert werden, wobei die Comonomeren (II) die folgende Formel aufweisen: 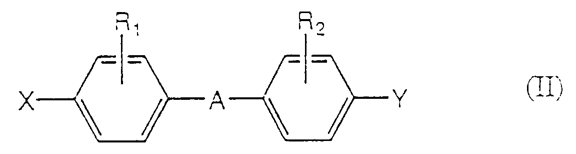 worin bedeuten:
X und Y, unabhängig voneinander, OCN oder 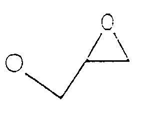
Y zusätzlich einen Alkyl-, Alkyloxy-, Alkyloxycarbonyl-oder Acyloxyrest einer kettenlänge von 1 bis 20 Kohlenstoffatomen, gegebenenfalls verzweigt,
A die Strukturelemente 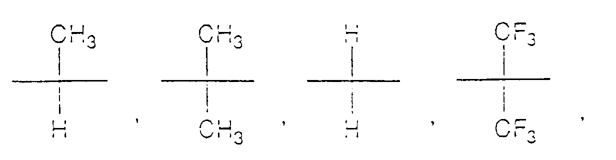 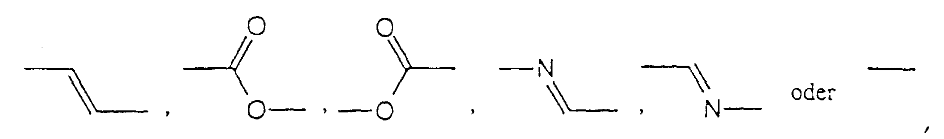 R₁ und R_{2,} unabhängig voneinander Wasserstoff, Halogen, insbesondere Chlor, Brom oder Fluor, oder einen Methyl- oder Ethylrest.

8. Verfahren nach Anspruch 7, dadurch gekennzeichnet, daß als Comonomer (II) 4,4'-Dicyanatophenylbenzoat, 4,4'-Dicyanatobiphenyl und/oder 2,2-Bis(4-cyanatophenyl)-propan verwendet werden.

9. Verfahren nach Anspruch 7 oder 8, dadurch gekennzeichnet, daß auf 100 Gew.-Teile Tris-(cyanato)-s-triazine der Formel (I) 30 bis 300 Gew.-Teile der Comonomere (II) entfallen.

10. Verfahren nach mindestens einem der Ansprüche 2 bis 9, dadurch gekennzeichnet, daß zusätzlich flüssigkristalline Cyanato-Comonomere (III) in Form von 1,4-Bis(cyanatobenzoyloxy)benzol und/oder 1,4-Bis(4-cyanatobenzoyloxy)-methylbenzol mitumgesetzt werden.

11. Verfahren nach mindestens einem der Ansprüche 3 bis 10, dadurch gekennzeichnet, daß die Polycyclotrimerisation während einer Zeitdauer von etwa 0,5 bis 2 h durchgeführt wird.

12. Verfahren nach mindestens einem der Ansprüche 3 bis 11, dadurch gekennzeichnet, daß eine Nachhärtung durchgeführt wird.

13. Verfahren nach mindestens einem der Ansprüche 2 bis 12, dadurch gekennzeichnet, daß die Eigenschaften des anisotropen duroplastischen Netzwerks, insbesondere die thermische Ausdehnung und die Zugfestigkeit in Orientierungsrichtung, durch Anlegen eines elektromagnetischen Feldes während der Polycyclotrimerisation gesteuert werden.

14. Anisotropes duroplastisches Netzwerk in Form eines Homo- oder Copolymerisats des Tris-(cyanato)-s-triazins der Formel (I) 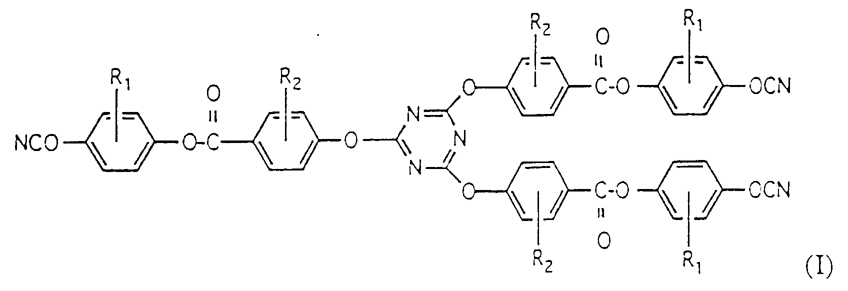 worin R₁ und R_{2,} jeweils unabhängig voneinander, Halogen, Wasserstoff oder einen Methyl- oder Ethyl-Rest bedeuten.

15. Anisotropes duroplastisches Netzwerk in Form eines Copolymerisats der Tris-(cyanato)-s-triazine der Formel (I) sowie der nicht-flüssigkristallinen Comonomeren (II) und/oder der flüssigkristallinen CyanatoComonomeren (III) gemäß den Definitionen nach den vorhergehenden Ansprüche 7 bis 11.

16. Anisotropes duroplastisches Netzwerk in Form eines Copolymerisats nach Anspruch 15, dadurch gekennzeichnet, daß darin auf 100 Gew.-Teile der Tris(cyanato)-s-triazine der Formel (I) 30 bis 300 Gew.-Teile der Comonomeren (II) und/oder bis zu 1000 Gew.-Teile der flüssigkristallinen Cyanato-Comonomeren (III) entfallen.

## Claims

1. Tris-(cyanato)-s-triazines having the formula (I) 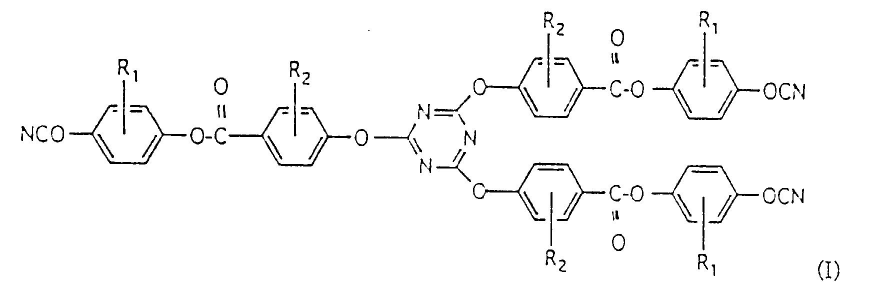 wherein R₁ and R₂ independently stand for halogen, hydrogen or a methyl or ethyl radical.

2. A method of preparing anisotropic thermosetting networks by polycyclotrimerisation of polycyanates in the melt, if required in the presence of catalysts, comonomers and other conventional additives, characterised in that polycyanates in the form of liquid crystal tris-(cyanato)-s-triazines having the formula (I) in claim 1 are polycyclotrimerised.

3. A method according to claim 2, characterised in that polycyclotrimerisation is carried out between 90 and 300°C.

4. A method according to claim 2 or 3, characterised in that polycyclotrimerisation is carried out in the presence of a catalyst.

5. A method according to claim 4, characterised in that metal naphthenates, octoates and/or acetyl acetonates are used as the catalyst.

6. A method according to any of claims 3 to 5, characterised in that the proportion is 0.01 to 3 parts by weight of catalyst per 100 parts by weight of the reactive constituents of the starting mixture.

7. A method according to at least one of claims 2 to 6, characterised in that tris-(cyanato)-s-triazines having the formula (I) are polycyclotrimerised together with non-liquid-crystalline comonomers (II), wherein the comonomers (II) have the following formula: 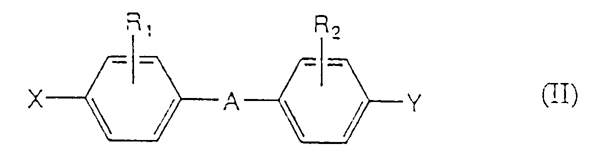 wherein
X and Y independently stand for OCN or 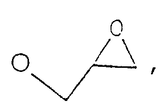
Y additionally stands for an alkyl, alkyloxy, alkyloxy carbonyl or acyloxy radical having a chain length of 1 to 20 carbon atoms, branched if required,
A denotes the structural elements: 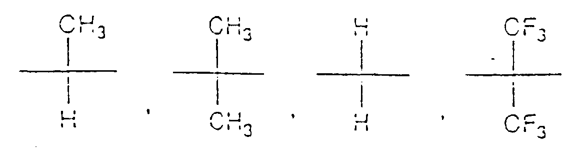 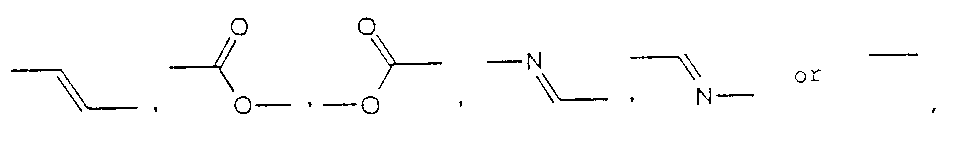 and R₁ and R₂ independently denote hydrogen, halogen, particularly chlorine, bromine or fluorine or a methyl or ethyl radical.

8. A method according to claim 7, characterised in that the comonomer (II) is 4,4'-dicyanatophenyl benzoate, 4,4'-dicyanatobiphenyl and/or 2,2-bis(4cyanatophenyl) propane.

9. A method according to claim 7 or 8, characterised in that the proportion is 30 to 300 parts by weight of the comonomers (II) per 100 parts by weight of tris(cyanato)-s-triazines having the formula (I).

10. A method according to at least one of claims 2 to 9, characterised in that liquid crystalline cyanato comonomers (III) in the form of 1,4bis (cyanatobenzoyloxy) benzene and/or 1,4-bis-4cyanatobenzoyloxy) methyl benzene are additionally reacted.

11. A method according to at least one of claims 3 to 10, characterised in that polycyclotrimerisation is carried out for about 0.5 to 2 hours.

12. A method according to at least one of claims 3 to 11, characterised in that after-curing is carried out.

13. A method according to at least one of claims 2 to 12, characterised in that the properties of the anisotropic thermosetting network, particularly the thermal expansion and tensile strength in the orientation direction, are controlled by applying an electromagnetic field during polycyclotrimerisation.

14. An anisotropic thermosetting network in the form of a homopolymer or copolymer of tris-(cyanato)-striazine having the formula (I) 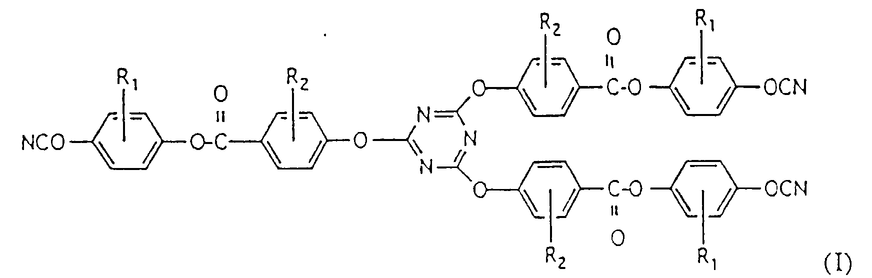 wherein R₁ and R₂ independently stand for halogen, hydrogen or a methyl or ethyl radical.

15. An anisotropic thermosetting network in the form of a copolymer of tris-(cyanato)-s-triazines having the formula (I) and of the non-liquid-crystalline comonomers (II) and/or the liquid crystalline cyanato comonomers (III) as defined in the preceding claims 7 to 11.

16. An anisotropic thermosetting network in the form of a copolymer according to claim 15, characterised in that the proportions therein are 30 to 300 parts by weight of comonomers (II) and/or up to 1000 parts by weight of the liquid crystalline cyanato comonomers (III) per 100 parts by weight of the tris-(cyanato)-striazines having the formula (I).

## Revendications

1. Tris-(cyanato)-s-triazines de formule (I) 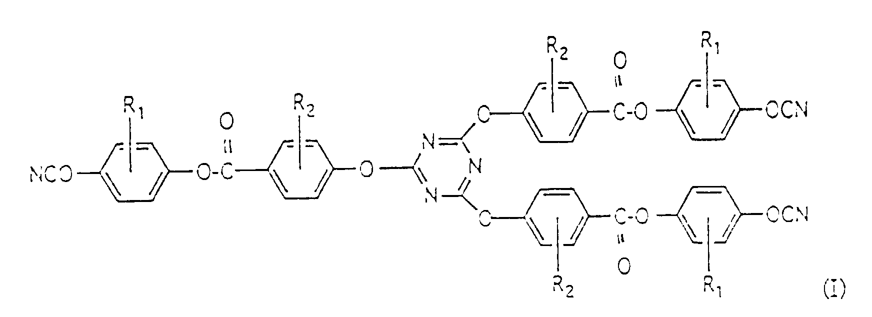 où R₁ et R₂ représentent, indépendamment l'un de l'autre, un halogène, l'hydrogène ou un reste méthyle ou éthyle.

2. Procédé pour la préparation de structures réticulaires thermoplastiques anisotropes par polycyclotrimérisation de polycyanates dans la masse fondue, éventuellement en présence de catalyseurs, de comonomères et d'autres additifs usuels, caractérisé en ce que l'on polycyclotrimérise des polycyanates sous forme de tris-(cyanato)-s-triazines cristaux liquides de formule (I) selon la revendication 1.

3. Procédé selon la revendication 2, caractérisé en ce que la polycyclotrimérisation est effectuée entre 90 et 300°C.

4. Procédé selon la revendication 2 ou 3, caractérisé en ce que la polycyclotrimérisation est effectuée en présence d'un catalyseur.

5. Procédé selon la revendication 4, caractérisé en ce que l'on utilise, comme catalyseur, les naphténates, octoates et/ou acétylacétonates métalliques.

6. Procédé selon l'une des revendications 3 à 5, caractérisé en ce que correspondent à 100 parties en poids des constituants réactifs du mélange de départ, 0,01 à 3 parties en poids de catalyseur.

7. Procédé selon au moins l'une des revendications 2 à 6, caractérisé en ce que l'on polycyclotrimérise les tris-(cyanato)-s-triazines de formule (I) associées à des comonomères (II) qui ne sont pas des cristaux liquides, les comonomères (II) ayant la formule suivante: 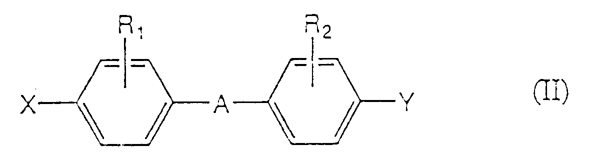 où
X et Y représentent, indépendamment l'un de l'autre, OCN
ou 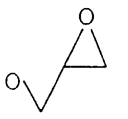
Y représente, en plus, un reste alkyle, alkyloxy, alkyloxycarbonyle ou acyloxy ayant une longueur de chaîne comprise entre 1 et 20 atomes de carbone, éventuellement modifiée,
A représente les éléments structuraux

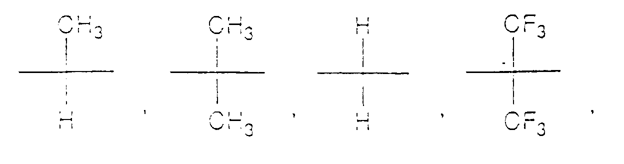

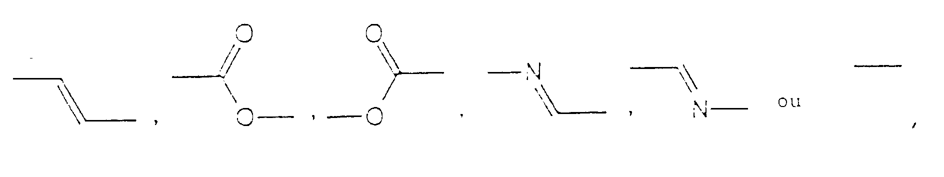

8. procédé selon la revendication 7, caractérisé en ce que l'on utilise, comme monomère (II), le benzoate de 4,4'-dicyanatophényle, le 4,4'dicyanatobiphényle et/ou le 2,2-bis(4-cyanatophényl)-propane.

9. procédé selon la revendication 7 ou 8, caractérisé en ce que correspondent à 100 parties en poids de tris(cyanato)-s-triazines de formule (I) 30 à 300 parties en poids de comonomères (II).

10. Procédé selon au moins l'une des revendications 2 à 9, caractérisé en ce que l'on fait réagir en même temps complémentairement des comonomères cyanato (III) cristaux liquides sous forme de 1,4-bis(cyanatobenzoyloxy)benzène et/ou de 1,4-bis(4-cyanatobenzoyloxy)méthylbenzène.

11. Procédé selon au moins l'une des revendications 3 à 10, caractérisé en ce que la polycyclotrimérisation est effectuée pendant une durée comprise entre environ 1/2 heure et environ 2 heures.

12. Procédé selon au moins l'une des revendications 3 à 11, caractérisé en ce que l'on effectue un traitement complémentaire de durcissement.

13. Procédé selon au moins l'une des revendications 2 à 12, caractérisé en ce que les propriétés de la structure réticulaire thermoplastique anisotrope, en particulier l'expansion thermique et la résistance à la traction dans la direction d'orientation, sont réglées par application d'un champ électromagnétique pendant la polycyclotrimérisation.

14. Structure réticulaire thermoplastique anisotrope sous forme d'un homo- ou copolymérisat de tris-(cyanato)-s-triazines de formule (I) 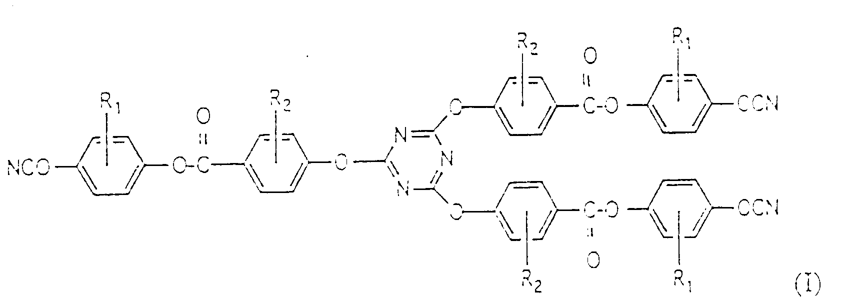 où R₁ et R₂ représentent, indépendamment l'un de l'autre, un halogène, l'hydrogène ou un reste méthyle ou éthyle.

15. Structure réticulaire thermoplastique anisotrope sous forme d'un copolymérisat de tris-(cyanato)-s-triazines de formule (I) et de comonomères (II) qui ne sont pas des cristaux liquides et/ou de comonomères cyanato (III) cristaux liquides selon les définitions données dans les revendications 7 à 11 précédentes.

16. Structure réticulaire thermoplastique anisotrope sous forme d'un copolymérisat selon la revendication 15, caractérisée en ce que dans un tel copolymérisat correspondent à 100 parties en poids de tris(cyanato)-s-triazines de formule (I) 30 à 300 parties en poids de comonomères (II) et/ou jusqu'à 1000 parties en poids de comonomères cyanato (IIi) cristaux liquides.
